# EUROPEAN PATENT APPLICATION

(11) **EP 4 032 560 A1**
(43) Date of publication of application: **27.07.2022**
(21) Application number: 21153325.2
(22) Date of filing: 25.01.2021
(51) Int. Cl.: A61L 27/18, A61L 27/34, A61L 27/50, A61L 27/54, A61L 27/58, A61L 33/00

(54) **IMPLANTABLE MATERIAL IN CONTACT WITH BLOOD AND USES THEREOF**

(71) Applicant: Centre National pour la Recherche Scientifique, 75794 Paris Cedex 16 (FR); UNIVERSITE DE STRASBOURG, 67000 Strasbourg (FR); Les Hôpitaux Universitaires de Genève, 1205 Geneva (CH); UNIVERSITE DE GENEVE, 1211 Geneva 4 (CH)
(72) Inventor: DECHER, Gero, 77694 Kehl-Marlen (DE); WALPOTH, Beat, 1222 Vésenaz (CH); FONTANA, Pierre, 1227 Carouge (CH); WITT, Maria, 80730-300 Curitiba (BR); DE VALENCE, Sarra, Oakland, CA California 94608 (US); TSCHOPP, Michel, 67600 Selestat (FR); FELIX, Olivier, 67810 Holtzheim (FR)
(74) Representative: reuteler & cie SA

(57) **Abstract**

The present invention is directed to materials compatible with blood, implantable devices comprising said material, methods of preparation of such material and medical devices coated therewith and uses thereof for anti-thrombotic and/or cell-proliferating aspects.

## Description

### Field of the Invention

The present invention relates to implantable materials to be put into contact with blood such as for example in implantable devices such as vascular grafts, shunts, patches, stents, valves and their preparation and applications thereof. In particular, the invention relates to biocompatible materials and implantable artificial devices useful for treatment of a human or animal by surgery or therapy (endoprostheses), especially of a cardiovascular disease, in particular of a cardiovascular ischaemic, degenerative or congenital disease, for example such as coronary artery disease or peripheral vascular disease or access surgery for dialysis patients, pediatric cardiovascular malformations, trauma and reparative surgery.

### Background of the Invention

Cardiovascular ischaemic diseases represent the leading cause of mortality in the western world with vascular grafts of small inner diameter (ID) remaining a challenge to material scientists. The major failure mechanisms associated to already commercially available grafts include early thrombosis/occlusions and lack of rapid and confluent endothelialization as well as late intimal hyperplasia which may lead to stenosis and occlusion.

This implies the need for the development of improved prosthetic vascular conduits that do not provoke pathological reactions after implantation. The graft should resemble native blood vessels with respect to biocompatibility, antithrombogenicity, shape, size, and mechanical properties, compliance, regenerative and growth potential as well as resistance to infections. The use of naturally derived materials such as collagen or decellularized tissue matrices bears the risk of immunologic reaction leading to early degradation resulting in aneurysms and rupture. The production of completely cell derived grafts is very time consuming and takes about 6 months. Therefore, in clinical practice surgeries using small caliber vascular grafts (<6 mm) are mainly performed with autologous arteries or veins; these can however be diseased or used for prior surgery and therefore there is a big need of shelf-ready small caliber vascular grafts. Synthetic polymers have the advantage that they can be produced on a large scale with controlled properties (physical and chemical) such as strength, degradation rate, and reproducible microstructure. Commercially available grafts are made of non-degradable polymers such as polyethylene terephthalate (PET), Dacron^{®}, or Expanded polytetrafluoroethylene (ePTFE) and are widely used as non-degradable synthetic vascular grafts.

They are successfully used to replace large diameter vessels, but they fail as small diameter prostheses (ID < 6 mm) needed for anastomosis to smaller vessels and/or microvascular surgery. Vascular grafts with a small inner diameter constitute a challenge to material scientists. They could be employed for treating cardiovascular ischaemic diseases such as coronary artery disease or peripheral vascular disease, which still represent the leading cause of mortality of non-transmissible diseases in the western world. Additionally, they may be required for the treating of end-stage renal disease in access surgery. When possible, by-pass surgeries are performed with autologous arteries or veins, since the results of synthetic vascular grafts show poor clinical results when used for small caliber grafts (ID < 6 mm). In contrast to large caliber grafts, prostheses with an inner diameter < 6 mm, lead to increased hemodynamic flow disturbances and thrombogenic complications. Thus, endothelialization of small caliber grafts should create a compatible interface for hemodynamic and functional processes. The major failure mechanisms are early thrombosis (cell surface interaction) and lack of rapid and confluent endothelialization. This failure is mainly a result of an unfavorable healing process with surface thrombogenicity due to lack of endothelial cells and anastomotic intimal hyperplasia caused by hemodynamic disturbances. Thereafter, intimal hyperplasia may lead to stenosis and occlusion often requiring a re-operation, or an amputation in the case of a peripheral location. All these limitations also apply to any devices (valves, VAD, etc.) implanted in contact with blood. As a result of the above, no synthetic grafts are used for coronary artery surgery. Therefore, the development of ideal small-diameter prostheses is a major challenge in vascular research.

Numerous surface coatings have been developed to improve blood compatibility of biomaterials and for more than thirty years, the anticoagulant drug heparin has been employed as a covalently immobilized surface coating on a variety of medical devices for improving blood compatibility as reviewed in Biran et al., 2016, Adv. Drug Deliv. Rev., 112, 12-23*.*

Numerous strategies of surface modification have been explored to further improve the blood compatibility (i.e. conjugation of heparin) of expanded-polytetrafluoroethylene (ePTFE), a so-called non-treatable surface and/or polycarbonate-urethane (PCU) surfaces, including chemical immobilization, physical adsorption, and plasma treatment. It was shown that PCU surface functionalization achieved using plasma treatment was significantly more effective compared to alternative methods such as aminolysis and physical adsorption via polydopamine coating to generate functional amine groups on the surface and that these functional amine groups grafted on the vascular graft surface could be then used for subsequent conjugation of heparin by reductive amination to allow end-point immobilization of heparin molecules on the graft surface (Qiu et al., 2017, Acta Biomater., 51, 138-147). Plasma treatment followed conjugation of heparin by reductive amination was described as advantageously providing not only higher surface density but also better stability and anti-thrombogenic activity and improved performance of the vascular grafts with respect to patency as well as early stages of endothelialization and graft integration.

Biodegradable polymers have also been investigated for their potential as a scaffold for making small diameter artery bypass grafts. Two methods of aminolysis on electrospun Poly-L-lactide-co-caprolactone (PLCL) microfiber vascular were compared: plasma treatments and Fmoc-PEG-diamine insertion method in view of end-point heparin conjugation to the PLCL scaffold in view of optimizing heparin density and bioactivity on the biodegradable electrospun PLCL microfiber vascular grafts (Hsieh et al., 2017, Biomed. Mater., 12, 6). The study concluded to superiority of plasma treatment regarding the initial heparin density, while inducing minimal polymer chain degradation, while the chemical introduction of amino groups for end-point conjugation of heparin (by the fmoc-PEG-diamine insertion method) led to partial fiber erosion, melting and structure disruption. However, plasma activation of the surface is quite aggressive toward the support material and frequently causes undesirable defects to the structure and mechanical properties of the implantable material even preventing further biomedical use of biodegradable materials such as PCL (polycaprolactone).

Gao et al., 2018, Regen. Biomater., 5, 105-114 have recently prepared small diameter vascular grafts based on a matrix of electrospun poly(ε-caprolactone) which was then coated with alternating layers (Layer-by-Layer (LbL) assembly) of selenium-containing catalyst-organoselenium modified polyethyleneimine and Heparin in order to combine the effects of heparinization and catalytic NO generation for stimulating tissue regeneration and vascularization and the vascular remodeling process. In this assembly, heparin is used as a polyanion for LbL-formation.

Thus, there is a need to find suitable material and/or coating that would allow the preparation of vascular grafts of small inner diameter enabling early transient anti-thrombotic properties that would prevent early thrombotic events and that would allow an early, rapid and confluent endothelialization of the graft.

### Summary of the invention

The present invention relates to the unexpected finding of a new biocompatible material suitable for *in vivo* tissue regeneration which allows the preparation of small calibre grafts (1-6 mm of internal diameter) useful for example in coronary artery bypass grafts (CABG) as described in Virk et al., 2019, Curr. Cardiol. Rep., 21, 36*.* This new material and coating presents mechanical properties suitable to match arterial compliance, with strain and stress superior to native arteries, and suitable to increase suture retention, prevent aneurysm formation and rupture that allows its use for vascular grafts, shunts, patches, endo-prosthesis, valves, and any implantable device in contact with blood. The process developed for the preparation of such a material presents the main advantage of leading to a material with highly reproducible structural properties compared to chemical or plasma functionalization and which is time effective over methods of preparation of grafts which needs *in vitro* cell work such as extra-cellular matrix formation based on cell-type, ingrowth and duration. (L'Heureux et al., 2006, Nat. Med., 12, 361-365)*.*

According to another aspect, a process according to the invention provides prolongued anticoagulation properties to the material. In particular, the material of the invention, when present in the luminal surface of a vascular patch or graft, leads to an unexpected long-lasting anti-thrombotic effect which prevents the early thrombus formation or occlusion. According to a particular embodiment, the material of the invention, when biodegradable, triggers a host response with cell ingrowth *a de novo* ECM (extra-cellular matrix) is formed *in vivo.*

Further, according to another particular aspect, when the multilayer polymer layer-by-layer (LbL) coating of the material of the invention comprises at least one pair of alternating layer of poly(allyl amine hydrochloride) (PAH) and poly(sodium 4-styrenesulfonate) (PSS) as an outer layer pair of said coating, the material of the invention further presents the advantage of providing advantageous cell attachment and growth properties by triggering a rapid endothelialisation due to a high affinity for the growing of endothelial cells onto its surface. Therefore, the material and the process of the invention allow achieving surprising properties for prolongued anticoagulation properties, while maintaining cell attracting properties.

Another particularly advantageous and non-obvious aspect is the finding of compositions and process steps allowing the above combination of functionalities on non-homogeneous substrate surfaces without using aggressive coupling reactions which would chemically modify the surface or which would alter the mechanical properties of the surface.

Therefore, the material and the process of the invention are expected to have beneficial effects for any biocompatible implantable material intended to be in contact with blood, when implanted, in particular for use in implantable devices.

An aspect of the invention provides a material compatible with blood comprising **(i)** a support comprising biocompatible non-woven fibers and **(ii)** a heparinized multilayer polymer layer-by-layer (LbL) coating of said biocompatible non-woven fibers wherein said multilayer polymer coating comprises at least one layer pair of an anionic polymer layer and a cationic polymer layer and the multilayer polymer layer-by-layer (LbL) coating is functionalized with heparin onto the outer layer pair of said multilayer polymer coating by end-point functionalization and wherein the anionic polymer is selected from heparin and poly(sodium 4-styrene sulfonate) (PSS) or a mixture thereof and the cationic polymer is selected from chitosan or an analogue thereof and poly(allyl amine hydrochloride) (PAH) or a mixture thereof.

Another aspect of the invention relates to an implantable device comprising a material according to the invention.

Another aspect of the invention relates to an extracorporeal circulation devices, oxygenators, hemodialysis/filtration membranes and catheters comprising a material according to the invention.

Another aspect of the invention relates to a process for preparing a material compatible with blood according to the invention.

Another aspect of the invention relates to a process for preparing an implantable device of the invention.

Another aspect of the invention relates to a method of treating a cardiovascular ischaemic disease such as coronary artery disease or peripheral vascular disease, pediatric cardiovascular malformations, a trauma or an organ deficiency such as end-stage renal disease (ESRD) or failure by reparative surgery in a subject in need thereof, said method comprising the implantation of an implantable device comprising a material of invention in contact with the blood of said subject.

Another aspect of the invention relates to a use of a material according to the invention or a method according to the invention for the preparation of an implantable device or an extracorporeal circulation device in contact with blood.

### Description of the figures

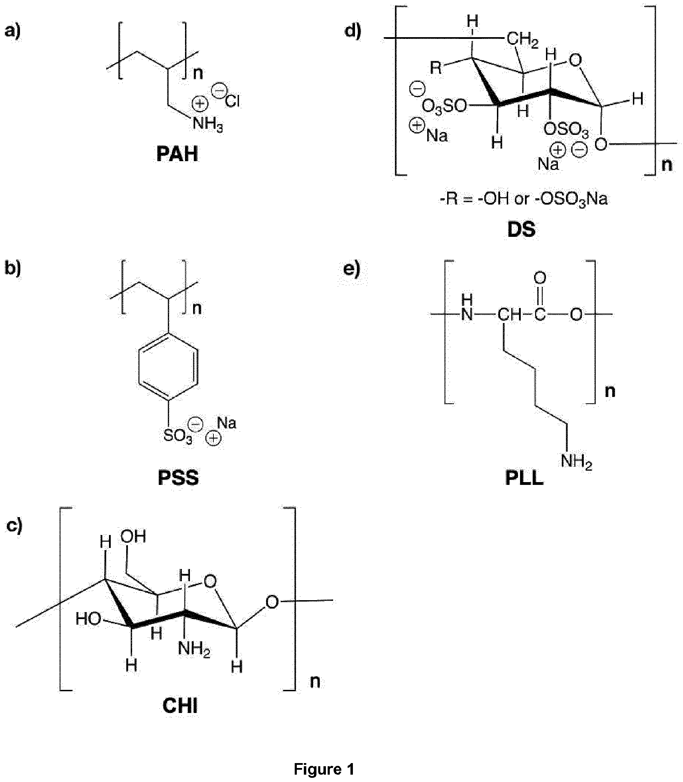

**Figure 1** shows the chemical structures of **(a)** poly(allylamine hydrochloride) (PAH), **(b)** poly(sodium 4-styrenesulfonate) (PSS), **(c)** fully deacylated chitosan (CHI), **(d)** dextran sulfate sodium salt (DS), and **(e)** poly(L-lysine) (PLL).

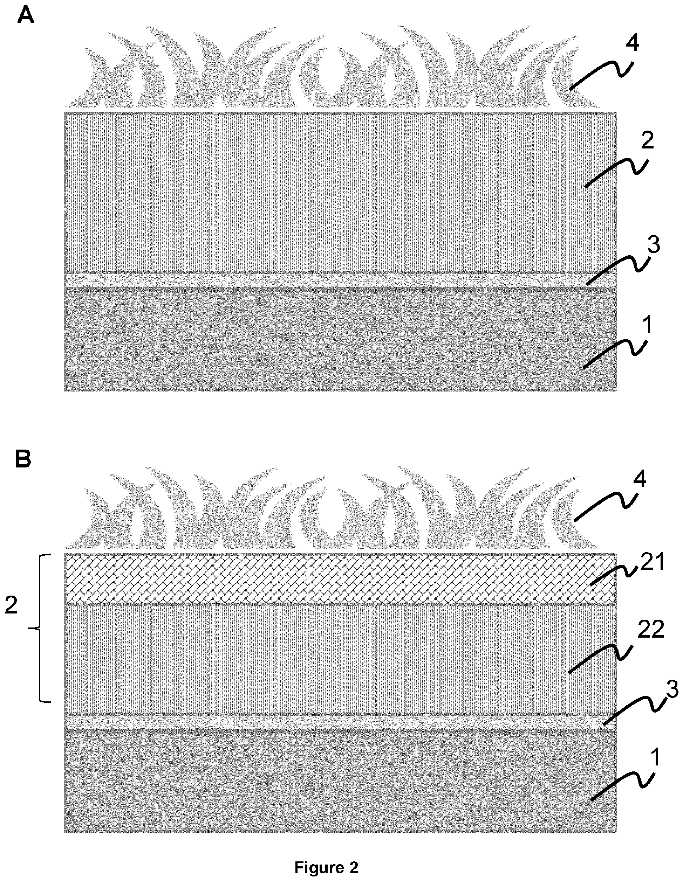

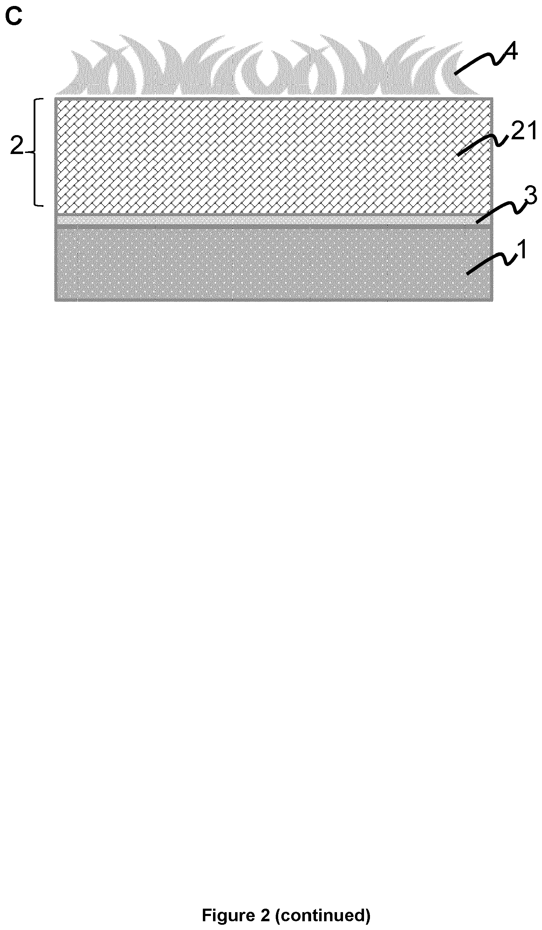

**Figure 2** is a schematic representation of the structure of a material compatible with blood of the invention. **A: material comprising (i)** a support comprising biocompatible non-woven fibers **(1); (ii)** heparinized multilayer polymer layer-by-layer (LbL) coating of the invention **(2);** an optional pre-coating deposit **(3),** wherein heparin **(4)** is end-point functionalized to the multilayer polymer coating **(2); B and C:** material comprising **i)** a support comprising biocompatible non-woven fibers **(1); (ii)** heparinized multilayer polymer layer-by-layer (LbL) coating of the invention **(2);** an optional pre-coating deposit **(3)** wherein the multilayer polymer coating **(2)** comprises at least one at least one pair of alternating layers of poly(allyl amine hydrochloride) (PAH) and poly(sodium 4-styrenesulfonate) (PSS) as an outer layer pair **(21)** which forms an heparinization substrate for end-point functionalization of heparin **(4),** the lower layers of the multilayer polymer coating being formed by layer pairs of other polymers of the invention **(22) (B) or** the lower layers of the multilayer polymer coating **(2)** being formed by layer pairs of the same polymers **(C).**

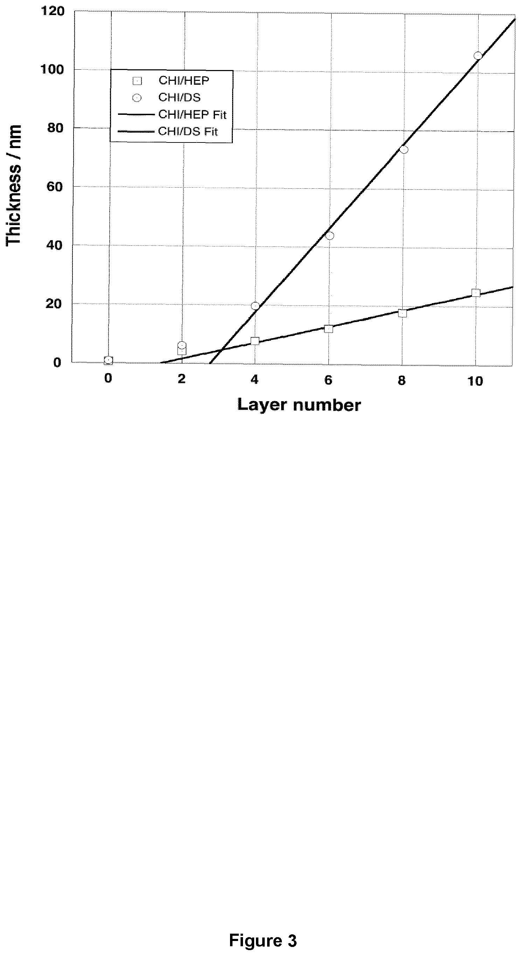

**Figure 3** represents the thickness (nm) of (CHI/HEP)ₙ and (CHI/DS)ₙ multilayer polymer coatings versus the number of LbL layers, i.e. the number of layer pairs of an anionic polymer layer and a cationic polymer layer deposited on a silicon wafer as described in Example 1. Note that slopes are taken at higher layer numbers as the thickness of first deposited layers is influenced by the substrate.

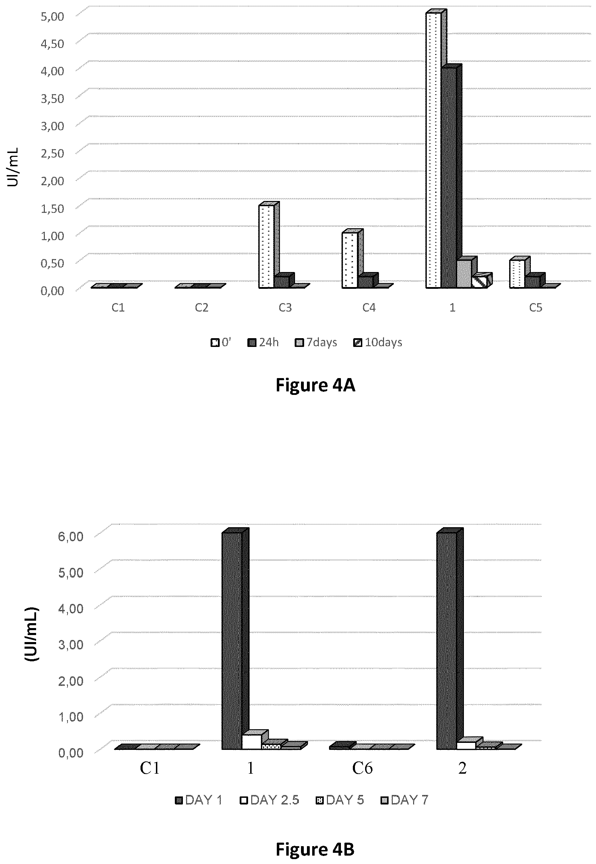

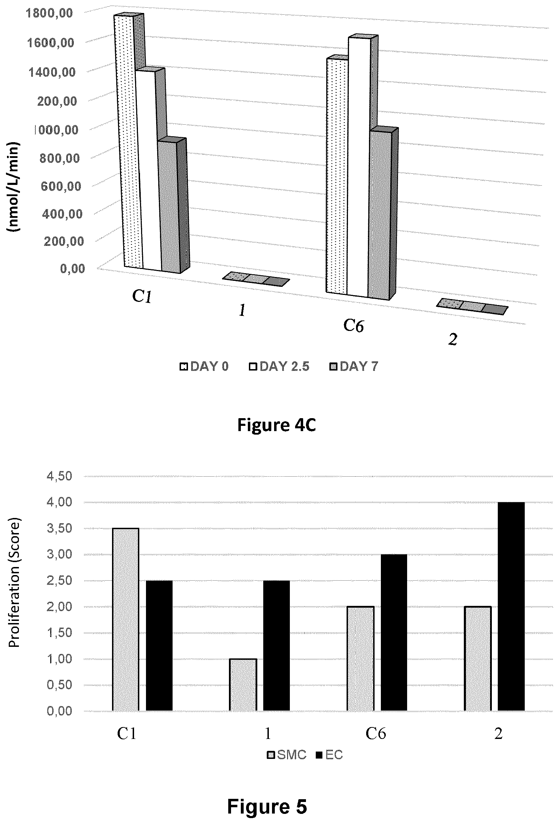

**Figure 4** shows the antithrombotic activity of various materials of the invention 1 and 2 compared to comparative coatings C1 to C6 expressed as Anti-Xa values (UI/ml) measured after 0h, 24h, 7 days and 10 days of incubation **(A)** or after 1, 2.5, 5 and 7 days of incubation **(B); (C)** shows thrombin generation inhibition (EPT: nmol/L/min) ability as measured according to the assay of Example 2b, after 0, 2.5 and 7 days of incubation.
**Figure 5** represents the semi-quantitative score (0-4) of cell proliferation for smooth muscle cells (SMC) **(grey)** and endothelial cells (EC) **(black)** from *in vitro* cell culture tested on materials of the invention 1 and 2 compared to comparative coatings C1 and C6 as described in Example 2c, after 5 days of incubation for the same samples as shown in **Figures 4B** and **C**. The higher score number of endothelial cells (EC) on the cell triggering coatings, the better is the endothelialisation.

### Detailed description

The term "heparin" refers to a heparin molecule, a fragment of a heparin molecule or a heparin derivative. Heparin is a polysaccharide belonging to the group of glycosaminoglycans bearing sulfonic groups distributed along its repeated units. Its structure contains a particular binding sequence that interacts with proteins in blood, especially anti-thrombin (AT) catalyzing the inhibition of thrombin (T) and factor Xa, for example. In an attempt to avoid the problems of intravenous administered heparin, this molecule has been immobilized most commonly to the material surface by three different methods; ionically (electrostatic interactions), multipoint attachment (covalent bond) and end-point functionalization (covalent bond). The attractive advantages of using this latter method include the orientation of the heparin molecules towards the bulk and the preservation of the active sites involved in the inhibition of thrombin generation. Heparin derivatives can be any functional or structural variation of heparin. Heparin is attached to the last LbL layer of the multilayer polymer layer-by-layer (LbL) coating of the invention.

The term "Layer-by-Layer (LbL) layer" defines a pair of an anionic polymer layer and a cationic polymer layer. According to a particular aspect, the number of LbL layers is from about 1 to about 8, typically from about 1 to about 5 (e.g. 4 or 5). According to a further particular embodiment, for use as a biodegradable graft, it is desirable to keep the number of LbL layers in a material of the invention as small as required for reaching the attempted functionality. Any additional layer will increase the stiffness of the graft and will also reduce its biodegradability. According to another particular embodiment, a multilayer polymer coating comprises one pair of alternating layers of poly(allyl amine hydrochloride) (PAH) and poly(sodium 4-styrenesulfonate) (PSS) as an outer layer pair.

The term "endothelial cell-attracting coating" refers to a coating that would allow cell attachment and trigger a rapid endothelialisation due to a high affinity for the growing of endothelial cells onto its surface. Endothelial cell-attracting property of a coating can be determined by conducting an assay as described herein or any other suitable assays such as longitudinal pre-clinical *in vivo* implantation studies. Examples of such as a suitable endothelial cell-attracting coating according to the invention comprises at least one pair of alternating layer of poly(allyl amine hydrochloride) (PAH) and poly(sodium 4-styrenesulfonate) (PSS) as an outer layer pair of said coating.

The term "artificial device" comprises an artificial device as defined in the present invention which is intended to be in contact with blood, such as an implantable device or an extracorporeal circulation device as well as catheters and other devices. Artificial devices include biodegradable and non-biodegradable devices. Implantable devices comprise vascular prostheses, grafts or shunts, cardiovascular patches, cardiovascular valves or leaflets, endoprostheses such as stents, ventricular assist devices, artificial hearts, intra-vascular devices such as filters, occlusion devices, and intra-vascular leads (pacemakers). Extracorporeal circulation devices include devices used for cardiopulmonary bypass, extracorporeal membrane oxygenation (ECMO) or other blood purification devices such as dialysis and their components. Biodegradable devices of the invention may further comprise a mesh, preferably non-biodegradable, to improve the mechanical stability during the degradation of the biodegradable device, in particular, a mesh improves flexibility and provides an anti-kink property to the implantable device, notably when inserted in a vascular graft.

The term "a support comprising biocompatible non-woven fibers" comprises materials that are suitable for grafts, patches or leaflets such as degradable and non-degradable polymers as described, but not limited to, in this application.

The expression "compatible with blood" refers to materials that can be used in contact with blood without causing harm, in particular that is thromboresistant. According to a particular embodiment, the blood compatibility can be assessed by an anti-thrombotic effect for a period of at least one week, or longer, typically from about one week to about a month, in standard assays such as those described herein.

As used herein, "treatment" and "treating" and the like generally mean obtaining a desired pharmacological and/or physical and/or physiological effect. The effect may be prophylactic in terms of preventing or partially preventing a disease, symptom or condition thereof and/or may be therapeutic in terms of a partial or complete cure of a disease, condition, symptom or adverse effect attributed to the disease. The term " treatment" as used herein covers any treatment of a disease in a mammal, particularly a human, and includes: (a) preventing the disease from occurring in a subject which may be predisposed to the disease but has not yet been diagnosed as having it; (b) inhibiting the disease, i.e., arresting its development; or relieving the disease, i.e., causing regression of the disease and/or its symptoms or conditions such as improvement or remediation of damage.

The term "subject" as used herein refers to mammals. For examples, mammals contemplated by the present invention include human, primates, and domesticated animals such as cattle, sheep, pigs, horses and particularly racehorses, laboratory rodents and the like.

The term "efficacy" of a treatment according to the invention can be measured based on changes in the course of disease in response to a use according to the invention. For example, the efficacy of a device according to the invention can be measured by improved experimental and clinical outcome with the implanted device/material of the invention.

### Material according to the invention

Referring to the figures, in particular first to **Figure 1A****,** a material compatible with blood comprises:
- a support comprising biocompatible non-woven fibers **(1),**
- a heparinized multilayer polymer layer-by-layer (LbL) coating of said biocompatible non-woven fibers **(2),**
wherein said multilayer polymer LbL coating comprises at least one layer pair of an anionic polymer layer and a cationic polymer layer and the multilayer polymer layer-by-layer (LbL) coating is functionalized with heparin onto the outer layer pair of said multilayer polymer by end-point functionalization and wherein the anionic polymer is selected from heparin and poly(sodium 4-styrene sulfonate) (PSS) or a mixture thereof and the cationic polymer is selected from chitosan or an analogue thereof and poly(allyl amine hydrochloride) (PAH) or a mixture thereof.

In another further embodiment of the invention, the material of the invention further comprises an optional pre-coating deposit **(3)** on the support comprising biocompatible non-woven fibers **(1),** said pre-coating deposit comprising an adhesion agent for the heparinized multilayer polymer LbL coating **(2).** In a particular embodiment, an adhesion agent can be a cationic polymer such as poly(ethylene imine) (PEI) or poly(lysine).

In another further embodiment of the invention, the material of the invention further comprises an optional endothelial cell-attracting coating of the multilayer polymer layer-by-layer (LbL) coating onto which heparin is functionalized by end-point functionalization. According to a particular aspect, an endothelial cell-attracting coating is particularly advantageous when used in combination with biodegradable non-woven fibers.

In one particular embodiment, the support comprising biocompatible non-woven fibers comprises or consists in biocompatible non-woven fibers such as microfibers and/or nanofibers or a mixture thereof.

In another embodiment, biocompatible non-woven fibers have a diameter of 0.1 to 10 µm, for example from 0.5 to 5 µm or from 1 to 5 µm.

In one particular embodiment, non-woven fibers form a tridimensional network or matrix with a certain porosity which is defined by the gap between the fibers.

Preferably, said non-woven fibers are non-oriented fibers, i.e. without any particular orientation along a specific axis or plane but can also be aligned using different electro-spinning parameters.

In one particular embodiment, said non-woven textile comprises or consists of several superimposed layers of fibers.

In one further particular embodiment, said non-woven textile comprises or consists of several superimposed layers with different porosities.

According to another particular embodiment, non-woven fibers according to the invention can be prepared as described in Pfeiffer et al., 2014, J. Biomed. Mater. Res., 102A, 4500-4509*.*

In another embodiment, biocompatible non-woven fibers are biodegradable. In particular, biodegradable biocompatible non-woven fibers can be found in a biodegradable polymer selected from the group consisting of degradable polycaprolactone (PCL), polyglycolic acid (PGA), polylactic acid (PLA), poly(lactic-co-glycolic acid) (PLGA), poly-L-lactide (PLLA), poly(glycerol sebacate) (PGS), polydioxanone (PDO, PDS) or poly-p-dioxanone or PGA-Poly-4-hydroxybutyrate (P4HB) copolymer, degradable polyurethanes fibers, and any combinations thereof as well as any other polymers used for biomedical applications such as described in Miranda et al., 2020, Antibiotics, 9, 174*.*

In a further particular embodiment, biodegradable biocompatible non-woven fibers comprise or consist of polycaprolactone (PCL) fibers.

In one specific embodiment, said fibers are ε-PCL nanofibers, and preferably electro-spun ε-polycaprolactone nanofibers. For example, those fibers can be obtained according to a process described in Nottelet et al., 2009, J. Biomed. Mater. Res., 89A, 865-875*.*

In one specific embodiment, said fibers are polycaprolactone having a molecular weight ranging from 10 to 200 kDA, preferably from 40 to 120 kDa, preferably from 60 to 100 kDa. In one embodiment, said non-woven textile comprises or consists of one or more layers having a porosity ranging from 0.8 to 12 µm. The porosity may be measured by scaffold porosity (e) or void fraction as described in de Valence et al., 2012, Acta Biomater.,8, 3914-3920.

In one embodiment, the multilayer polymer coating is a Layer-by-Layer (LbL) deposited multilayer coating. For example, LbL deposited multilayer coating can be made as described in Picart et al., 2015 in: Layer-by-Layer Films for Biomedical Applications, C. Picart, F. Caruso, and J.-C. Voegel (Eds), Wiley-VCH: Weinheim, Germany*;* El-Khouri et al., 2011 in: Functional Polymeric Ultrathin Films, R. Advincula and W. Knoll (Eds.) Wiley-VCH: Weinheim, Germany*.* Typically, the multilayer polymer coating has a total thickness from about 0.1 nm to about 500 nm, typically from about 1 nm to about 500 nm, in particular from 1 nm to about 100 nm, each layer pair having a thickness from about 0.2 nm to about 50 nm (e.g. from about 1 to 25 nm), as measured for example by ellipsometry on silicon wafer as shown in **Figure 3****.**

In one embodiment, the anionic polymer or cationic polymers have a molecular weight (*M̅_̅{̅w̅}̅*) ranging from 5 kDa to 1 MDa, for example from 7.5 kDa to about 1000 kDa such as from about 5 to 200 kDa, such as from about 5 to 75 kDa (e.g. about 7.5 kDa) or from about 15 kDa to 75 kDa. According to a particular embodiment, the anionic polymer cationic polymers suitable according to the invention have a molecular weight below 75 kDa.

In one particular embodiment, the anionic polymer or cationic polymers have a molecular weight (*M̅_̅{̅w̅}̅*) ranging from 7.5 kDa to 75 kDa.

In one embodiment, the anionic polymer is selected from heparin and poly(sodium 4-styrenesulfonate) (PSS) or a mixture thereof.

In another embodiment, the cationic polymer is selected from chitosan or an analogue thereof, and poly(allyl amine hydrochloride) (PAH) or a mixture thereof.

In one further embodiment, the anionic polymer is selected from heparin and poly(sodium 4-styrenesulfonate) (PSS) and the cationic polymer is selected from chitosan or an analogue thereof and poly(allyl amine hydrochloride) (PAH).

In one particular embodiment, an anionic polymer is heparin.

In one particular embodiment, an anionic polymer is poly(sodium 4-styrenesulfonate) (PSS). In one particular embodiment, a cationic polymer is chitosan or an analogue thereof such as N-alkylated chitosan such as tri-methyl chitosan.

In one particular embodiment, chitosan has a molecular weight from about 7.5 kDa to 75 kDa. In another particular embodiment, chitosan has a deacetylation degree of at least 80%, preferably of at least 85 %, typically from about 80 to about 95%.

In another particular embodiment, tri-methyl chitosan is prepared as described in Mourya et al., 2009, J. Mater. Sci. Mater. Med., 20, 1057- 79 *or* Malik et al., 2018, Int. J. Nanomedicine, 13, 7959-7970 *or* Kulkarni et al., 2017, Carbohydr. Polym., 157, 875-902*.*

In one particular embodiment, anionic polymer is poly(allyl amine hydrochloride) (PAH).

In one further particular embodiment, the multilayer polymer LbL coating comprises from about three layer pair to about eight layer pairs of an anionic polymer layer and a cationic polymer layer according to the invention, wherein said layer pairs are arranged such that the anionic polymer and the cationic polymer layers are alternating in the multilayer polymer coating.

In one further particular embodiment, the multilayer polymer LbL coating of the material of the invention comprises at least one pair of alternating layer of poly(allyl amine hydrochloride) (PAH) and poly(sodium 4-styrenesulfonate) (PSS) as an outer layer pair of said coating which is functionalized with end-point functionalized heparin.

According to a further particular embodiment, the multilayer polymer LbL coating comprises from about one layer pair to about eight layer pairs of an anionic polymer layer and a cationic polymer layer, wherein said anionic polymer is heparin and the said cationic polymer layer is selected from chitosan or an analogue thereof and a pair of alternating layer of poly(allyl amine hydrochloride) (PAH) and poly(sodium 4-styrenesulfonate) (PSS) as an outer layer pair of said coating which is functionalized with end-point functionalized heparin.

In one particular embodiment, the support comprising biocompatible non-woven fibers, is first coated by a pre-coating deposit comprising an adhesion agent for the multilayer polymer coating. In a particular embodiment, an adhesion agent is a cationic polymer such as poly(ethylene imine) (PEI), poly(lysine) and the like.

Referring to the figures, in particular first to **Figure 2****,** the heparinized multilayer polymer LbL coating **(2)** is obtained by functionalization of heparin **(4)** onto the outer layer of said multilayer polymer coating **(2)** by end-point functionalization, independently of the fact that heparin may be used as an anionic polymer in the multilayer polymer LbL coating **(2).**

Various end-point functionalization strategies for heparin are known for example such as described in Biran et al., 2016, Adv. Drug Deliv. Rev., 112, 12-23*.*

According to a further particular embodiment, heparin is functionalized onto the outer layer pair of said multilayer polymer LbL coating through end-point attachment (EPA) where the reducing end of heparin (generally first partially depolymerized by nitrous acid deamination) is covalently bound to the amino functional groups of the last layer of the multilayer polymer LbL coating by reductive amination.

According to a further particular embodiment, heparin is functionalized onto the outer layer pair of said multilayer polymer LbL coating through end-point conjugation (EPC), in particular where heparin is bound to the amino functional groups of the last layer of the multilayer polymer LbL such as described in *Hsieh et al., 2017, supra.*

According to a further particular embodiment, heparin is functionalized after diazotation of heparin for providing highly reactive aldehyde groups on heparin before functionalization onto the polymer coating.

According to a further particular embodiment, end-point conjugation of heparin allows to provide a sufficient access of the endothelial cells to the outer layer of the LbL coating, in particular of the cell-attracting coating of the multilayer polymer layer-by-layer (LbL) coating onto which heparin is functionalized by end-point conjugation.

Referring to the figures, in particular first to **Figures 2B** and **2C****,** in another particular embodiment, the multilayer polymer LbL coating of the support comprising biocompatible non-woven fibers comprises at least one pair of alternating layer of poly(allyl amine hydrochloride) (PAH) and poly(sodium 4-styrenesulfonate) (PSS) as an outer layer pair **(21)** and forms an heparinization substrate for end-point functionalization of the heparin **(4).** In this case, heparin **(4)** is end-point functionalized to the multilayer polymer coating **(2),** the lower layers of the multilayer polymer coating being formed by layer pairs of either the same polymers **(****Figure 2C****)** or preferably of other polymers described herein **((22) in** **Figure 2B****).**

According to a further particular embodiment, the multilayer polymer LbL coating of the support comprising biocompatible non-woven fibers is such that the support (optionally pre-coated with a pre-coating agent), is first coated with the anionic polymer layer of the first LbL layer pair.

In another particular embodiment, the multilayer polymer LbL coating of the support comprises several layer pairs of an anionic polymer layer and a cationic polymer layer, wherein the anionic polymer layers can be made of the same or different anionic polymer or cationic polymers between the different layer pairs.

In another particular embodiment, the multilayer polymer LbL coating of the support comprises from about 3 to about 8, in particular 3 to 5 alternating anionic/cationic polymer layer pairs, wherein the polymer layer pairs are selected from heparin/chitosan or an analogue thereof layer pairs and PAH/PSS layer pairs, or a combination thereof.

In another particular embodiment, the multilayer polymer coating of the implantable material support comprises from about 3 to about 8, such as from about 3 to 7, such as from about 3 to about 5, in particular 4 to 5, layer pairs of an anionic polymer layer and a cationic polymer layer wherein the anionic polymer is heparin and the cationic polymer is chitosan or an analogue thereof.

In another particular embodiment, the multilayer polymer LbL coating of the support comprises from about 3 to about 8 such as from about 3 to 7 such as from about 3 to 4, in particular from about 4 to 5 layer pairs of an anionic polymer layer and a cationic polymer layer, wherein the anionic polymer is PSS and the cationic polymer is PAH

In another particular embodiment, the multilayer polymer LbL coating of the support comprises from about 3 to about 7 such as from about 3 to about 5 layer pairs (e.g. 3, 4 or 5) of an anionic polymer layer and a cationic polymer layer, wherein the anionic polymer is heparin (HEP) and the cationic polymer is chitosan (CHI) and an outer polymer layer pair(s) consisting in from about 1 to about 2 layer pairs of an anionic polymer layer and a cationic polymer layer wherein the anionic polymer is PSS and the cationic polymer is PAH and wherein heparin is end-point functionalized to the outer polymer layer pair(s) PSS/PAH

In one particular embodiment, the thickness increment per layer pair from the multilayer polymer LbL coating ranges from 1 to 20 nm, in a multilayer polymer coating comprising or consisting of (HEP/CH) layer pair(s) and in a multilayer polymer coating comprising or consisting of (PSS/PAH) layer pair(s).

According to an advantageous aspect of the invention, the thickness of the multilayer polymer LbL coating can be adjusted by the selection of particular polymer layer pair.

In another further particular embodiment, the material according to the invention comprises biocompatible non-woven fibers coated with a system comprising a multilayer (LbL) polymer assembly from the following group:
- (optional adhesion agent)-(Hep/Chi)s-Hep, wherein the last LbL layer of (Hep/Chi)₅ is functionalized with Heparin by end-point conjugation; and
- (optional adhesion agent)-(PSS/PAH)₄-Hep, wherein the last LbL layer of -(PSS/PAH)₄ is functionalized with Heparin by end-point conjugation.

According to a particular aspect, an adhesion agent is present.

According to another particular aspect, the adhesion agent is PEI.

### Preparation of a material according to the invention

According to a particular embodiment, is provided a method for preparing a material compatible with blood comprising the steps of:
- providing biocompatible non-woven fibers as a support;
- coating said support with a multi-layered polymer LbL coating;
wherein the multilayer polymer LbL coating comprises at least one layer pair of an anionic polymer layer and a cationic polymer layer and wherein the anionic polymer is selected from heparin and poly(sodium 4-styrene sulfonate) (PSS) or a mixture thereof and the cationic polymer is selected from chitosan or an analogue thereof and poly(allyl amine hydrochloride) (PAH) or a mixture thereof.
- functionalizing the outer layer pair of said multilayer polymer layer-by-layer (LbL) coating with heparin by end-point functionalization.

According to a particular embodiment, the implantable material is in the form of biocompatible non-woven fibers provided as a support by electrospinning.

According to another particular embodiment, the biocompatible non-woven fibers are spun to form a support in the form of a tubular structure forming a lumen with a wall forming a luminal surface.

According to a particular embodiment, the support of non-woven fibers is subjected to a pretreatment by immersion in an alcoholic solution for about 10 min then in ultra-pure water for about 10 min, then drying before coating (e.g. in a solution containing ethanol such as ethanol from about 100% to about 95%).

According to another particular embodiment, the method for preparing a material compatible with blood further comprises an optional pre-coating step of providing a pre-coating deposit of an adhesion agent onto the surface of the implantable material before the multi-layered polymer coating.

According to a particular embodiment, a pre-coating step with an adhesion agent for the multi-layered polymer LbL coating is used advantageous when the first polymer layer of the first layer pair is a cationic polymer.

According to another particular embodiment a pre-coating step with an adhesion agent for the multi-layered polymer coating is advantageous when the implantable material is PCL.

According to another particular embodiment, is provided a method for preparing a material compatible with blood according to the invention wherein the multi-layered polymer LbL coating is applied onto the biocompatible non-woven fibers (either directly or on the pre-coated biocompatible non-woven fibers) by Layer-by-Layer (LbL) deposition.

According to another particular embodiment, is provided a method for preparing a material compatible with blood according to the invention wherein heparin is functionalized by end-point functionalization to the multi-layered polymer coating.

According to another particular embodiment, is provided a method for preparing a material compatible with blood according to the invention wherein the multilayer polymer LbL coating of the biocompatible non-woven fibers comprises at least one pair of alternating layer of poly(allyl amine hydrochloride) (PAH) and poly(sodium 4-styrenesulfonate) (PSS) as an outer layer pair and forms an heparinization substrate for end-point functionalization of the heparin. According to another particular embodiment, the heparinized multilayer polymer coating according to the invention has anti-thrombotic and/or cell growth properties and therefore can be used on any support that is intended for blood contact for any in vitro, animal or human applications.

### Artificial device of the invention

According to another particular embodiment, the substrate of a material of the invention can be an implantable or non-implantable medical device in contact with blood, in particular a stable or biodegradable implantable artificial device in contact with blood, such as those defined herein.

According to a particular embodiment, the implantable medical devices according to the invention have antithrombotic properties.

According to a particular embodiment, the implantable medical devices according to the invention have anti-coagulant properties.

According to a particular embodiment, the implantable medical devices according to the invention do not need a pre-endothelialiation step before implantation.

According to a particular embodiment, is provided implantable medical device suitable for contact with blood, said device comprising a surface (e.g. a wall) comprising biocompatible non-woven fibers, wherein said surface is coated with multilayer polymer coating according to the invention.

According to a particular embodiment, is provided implantable medical device suitable for contact with blood, wherein the surface comprising a biodegradable and biocompatible non-woven fibers is formed by electrospun biocompatible non-woven fibers.

In one embodiment, is provided a biodegradable implantable medical device, in particular a vascular graft having a tubular structure forming a lumen and wherein the wall of said device forms the luminal surface.

According to a particular embodiment, is provided implantable medical device suitable for contact with blood, wherein said implantable medical device is an artificial blood vessel.

In one embodiment, said device is a biodegradable vascular graft having a tubular structure forming a lumen and wherein the wall of said device forms the luminal surface, and wherein said biodegradable vascular graft is of any size, diameter or architecture (straight or bifurcated) but in particular having an inner diameter of less than 6 mm (ID<6 mm), typically from about 1 to about 6 mm of internal diameter.

In one specific embodiment, the biodegradable vascular graft is a coronary artery vascular graft. Advantageously, the implantable artificial device such as a biodegradable vascular graft according to the invention is a permanent or temporary intracorporeal medical device.

In one embodiment, the implantable artificial device according to the invention is selected from the group consisting of stents including bifurcated stents, balloon expandable stents, self-expanding stents, stent-grafts including bifurcated stent-grafts, grafts including vascular grafts, bifurcated grafts, embolic filters, artificial blood vessels, drug delivery devices/balloons, patches, intra-vascular occlusion devices, CNS shunts (e.g., a ventriculopleural shunt, a VA shunt, or a VP shunt), ventricular peritoneal shunts, ventricular atrial shunts, portosystemic shunts and shunts for ascites, cardiac valve leaflets, shunts for pediatric cardiac surgery, vascular grafts and shunts for access surgery for dialysis. Furthermore, biodegradable implantable medical device according to the invention also artificial hearts, LVAD or non-implantable extra-corporeal support (CPB/ECMO) or blood purification machines and their catheters.

### Preparation of an artificial device of the invention

According to a particular embodiment, is provided a method for the preparation of an implantable device comprising the following steps:
- providing an implantable device or a part thereof (e.g. a wall of said implantable device) comprising biocompatible non-woven fibers as support;
- coating said support with a multi-layered polymer LbL coating according to the invention. According to a particular embodiment, is provided a method for preparing an implantable device compatible with blood, wherein said process comprises the steps of:
   - forming a wall by electrospinning biocompatible non-woven fibers;
   - coating said wall with multilayer polymer coating by Layer-by-Layer (LbL) deposition said wherein multilayer polymer coating comprises at least one layer pair of an anionic polymer layer and a cationic polymer layer according to the invention;
   - functionalizing said multi-layered polymer LbL coating on its outer layer with heparin by end-point functionalization.

According to a particular embodiment, the implantable device or a part thereof is provided as support by forming a wall by electrospinning biocompatible non-woven fibers.

According to a particular embodiment, is provided a method for the preparation of an implantable device of the invention wherein a non-degradable or degradable mesh is introduced into a non or fully biodegradable implantable device (e.g. artificial) in contact with blood, and in particular a fully biodegradable vascular graft. According to this specific embodiment, when the fully degradable vascular graft is implanted in the arterial or venous circulation, or any other tubular structure, the non-degradable or degradable mesh would prevent a rupture or dilatation or kinking of the wall of the graft in the event of insufficient vascular regeneration after full polymer degradation. The mesh improves the mechanical stability of the graft before, during, and after degradation. Accordingly, the invention relates to an artificial device comprising a fully biodegradable implantable artificial device as defined in the present invention and a non-degradable or degradable mesh.

### Patients

In an embodiment, patients according to the invention are subjects suffering or at risk of suffering from an ischaemic, degenerative or congenital cardiovascular disease, a trauma (e.g. neurological, peripheral, cardiac, vascular or orthopaedal trauma) or an organ deficiency or failure such as end-stage renal disease (ESRD).

In a further embodiment, patients according to the invention are subjects undergoing reconstructive surgery such as vascular reconstructive surgery.

### Uses according to the invention

According to a particular embodiment, the material, process and methods of the invention are useful for the treatment of a cardiovascular disease by surgery or therapy, in particular of a cardiovascular ischaemic disease, for example such as coronary artery disease, peripheral vascular disease or access surgery for dialysis, or graft or shunt applications in pediatric patients, pediatric cardiovascular malformations, trauma or reparative surgery.

According to a particular aspect, biocompatible materials according to the invention may be useful for the preparation of biodegradable implantable artificial devices such as vascular grafts useful for treatment of a human or animal by surgery or therapy (endoprostheses), especially of a cardiovascular disease, in particular of a cardiovascular ischaemic disease, for example such as coronary artery disease or peripheral vascular disease or access surgery for dialysis patients, pediatric cardiovascular malformations, trauma and reparative surgery.

Examples illustrating the invention will be described hereinafter in a more detailed manner and by reference to the embodiments represented in the Figures.

### EXAMPLES

### Example 1: Preparation of a vascular grafts with a material of the invention

The material of the invention was applied onto a substrate useful as an implantable vascular graft.

All reagents were used as received without any additional purification steps.

Poly(allylamine hydrochloride) (PAH, ≈ 50,000 g/mol), poly(sodium 4-styrenesulfonate) (PSS, ≈ 70,000 g/mol) and dextran sulfate sodium salt from Leuconostoc ssp. (DS, ≥ 500 000 g/mol) were purchased from Sigma-Aldrich (St. Quentin Fallavier, France), poly(L-lysine) hydrobromide (PLL, ≈15,000-30,000 g/mol) and chitosan (CHI, low molecular weight, 75-85 % deacetylated) were purchased from Sigma-Aldrich (Schnelldorf, Germany). Branched poly(ethylene imine) (PEI, Lupasol WF, ≈ 25,000 g/mol) was purchased from BASF (Ludwigshafen, Germany). Heparin sodium salt from porcine intestinal mucosa (>180 USP units/mg, reference H4784 from SIGMA) was used.

The solution of PEI was prepared at a concentration of 2.5 mg/mL in ultrapure water. The polyelectrolyte solutions used for the assembly of LbL-films can be divided into three distinct groups based on the polyanion used, HEP, DS or PSS.

For HEP containing LbL-films the polymer solutions were prepared in 0.15 M sodium chloride solution at a concentration of 1.0 mg/mL except for CHI. Chitosan was dissolved in a solution of acetic acid (0.5M) and in 0.15 M NaCl to obtain a final concentration of 2.0 mg/mL. HEP solution at 1 mg/mL was prepared using an acetate buffer (pH 4).

For DS-based LbL-films, an aqueous solution of DS (pH≈ 6.5) was prepared in 1 M NaCl at a polymer concentration of 1 mg/mL. Chitosan was dissolved in a solution of acetic acid (0.5M) and in 1 M NaCl to obtain a final concentration of 2.0 mg/mL. PLL solution was prepared at a concentration of 1 mg/mL and at 1M NaCl.

For PSS containing LbL films the polymer solutions were prepared in 0.15 M sodium chloride solution at a concentration of 1.0 mg/mL.

No final adjustment of pH values was carried out for any of the polyelectrolyte solutions.

The mixture DS:HEP 1:10 was prepared in an acetate buffer at 0.15 M NaCl considering the molar ratio (number of chains) for each component per milliliter, keeping a final polymer concentration of 1 mg/mL (77.3 mg of DS and 23.2 mg of HEP dissolved in 100 mL of buffer).

### a) providing a support comprising biocompatible non-woven fibers

Biocompatible non-woven fibers were used as support of a coating of the invention. For this purpose, ε-polycaprolactone (PCL) micro and nano-fiber scaffolds were prepared by electrospinning. In brief, a solution of 15% PCL (80,000 Da, Sigma) in CHCl₃/EtOH (70% v/v) was charged at 20 kV (Spellman High Voltage Electronics Ltd, Pulborough, West Sussex, UK) and ejected through a stainless steel needle at a continuous rate of 12 mL/h by means of a syringe pump (Fresenius Vial SA, Brezins, France). The polymer fibers were collected at a 20 cm needle-collector distance on a grounded, rotating (4,500 rpm) and translating (200 movements min⁻¹ and 4 cm amplitude) stainless steel mandrel (2 mm, or smaller or larger diameter), mounted on a homemade stand. After electrospinning, the grafts were placed in vacuum overnight to remove any residual solvent. The average size and distribution of such PCL fibers were measured in previous studies corresponding to an average fiber diameter of 2.2 ± 0.6 µm.

The resulting fiber meshes, here referred as "grafts" or "patches", were used as the bioartificial surface mimicking vascular grafts used in the cardiovascular surgical implants. A short pretreatment of the non-woven fiber support (e.g. graft/patches) was conducted by dipping those in ethanol for 10 min and rinsed for 10 min in ultra-pure water and dried. Sterilization before implantation was performed using gamma with a minimal dose of 25 kGy and a maximum dose of 32 kGy.

### b) Optionally coating the support with a pre-coating deposit comprising an adhesion agent

When a pre-coating deposit of an adhesion agent is used, the PEI solution is coated onto the non-woven fiber substrate obtained above by dipping for 15 min and rinsing three times two minutes in ultra-pure water followed by drying.

### c) Coating the support (pre-coated or not with an adhesion agent) with a multilayer polymer LbL coating comprising at least one layer pair of an anionic polymer layer and a cationic polymer layer of the invention

Samples of approximately 3 cm x 6 cm of the PCL-patches obtained above were coated by dipping the substrate into the solution. The time for polymer deposition and rinsing steps was about 1 min in the polyelectrolyte solution followed by 3 x 8 sec rinsing in ultra-pure water. The following materials of the invention comprising LbL film combinations of the invention **(LbL systems 1 to 2)** as well as comparative coatings **C1** to **C6** are prepared on the biocompatible non-woven fiber surface of different devices as listed in Table 1 below:

**Table 1**

| **Coating materials** | **Coating Type** |
|---|---|
| 1 (of the invention) | PEI-(Hep/Chi)₅-Hep endpoint |
| 2 (of the invention) | PEI-(PSS/PAH)₄-Hep endpoint |
| C1 | Bare patch (no coating) |
| C2 | PEI-(DS/Chi)₅-DS |
| C3 | PEI-(DS:Hep/Chi)₅-(DS:Hep) |
| C4 | PEI-(DS/PEI)₅-Hep endpoint |
| C5 | PEI-(DS/PLL)₅-Hep endpoint |
| C6 | PEI-(PS S/PAH)₄ |

### d) Heparinization of the multi-layered polymer coating on the last LbL layer

A heparinized multilayer polymer LbL coating is obtained by functionalization of heparin onto the outer layer of said multilayer polymer coating, independently of the fact that heparin is incorporated as anionic polymer of the multilayer polymer LbL coating or not.

Heparinization can be carried out by various known techniques. In a preferred aspect, heparin molecules are attached to the surface of multi-layered polymer coating using end-point conjugation as described in *Hsieh et al., 2017, supra.*

For heparin end-point conjugation, the material obtained under the preceding step is immersed in a Hep EPC solution (0.02 M sodium monobasic phosphate, 0.2 M sodium chloride, and 3 mg mL⁻¹ sodium cyanoborohydride) for 24 hours and stirred using an orbital shaker (200 rpm) then rinsed 3 x 2 min in ultra-pure water and dried.

According to a particular aspect, an advantageous heparinization substrate for heparin end-point conjugation is formed by at least one outer layer pair comprising poly(allyl amine hydrochloride) (PAH) and poly(allyl amine hydrochloride) (PSS).

The amounts of heparin attached to the multi-layered polymer coating was determined using a carbazole assay as described in US 4,613,665 which was about 9.6 µg/cm².

These results suggest that the multi-layered polymer LbL coating present sufficient amount of primary amino groups toward the bulk to react with heparin (aldehyde end groups) molecules efficiently.

### Example 2: characterization of the coated implants with the material of the invention

The implants obtained under Example 1 were tested as follows:

### a) Stability of grafts

Blood medium is simulated using an immersion into 0.15M NaCl aqueous solution at room temperature or 37°C for at least 24 h up to 7 and/or 10 days considering for additional events, which would include mainly protein adsorption on the top of the coating to evaluate the stability of such films under similar conditions as of the antithrombotic experiments. The total film thickness (nm) over time (h) was evaluated by ellipsometry on silicon wafer and the multi-layered polymer coating thickness preserved more than 90 % of the initial values suggesting that film integrity was kept for more than seven days under these conditions.

This stability suggests that it could help the host body to go through the endothelialization step safe from clot formation, and the complications that arises from it.

Further, it has been observed that γ sterilization (25KGy) and storage (refrigerated up to nine months) does not alter the stability results regarding the vascular grafts.

### b) Antithrombotic properties

The antithrombotic performance of the vascular grafts was assessed *in vitro* using two complementary methods.

First, the amount of anticoagulant that was released from the graft when incubated with human plasma was evaluated. For this purpose, grafts were put into tubes and incubated with 700 µL of pooled human plasma (CRYOcheck, PrecisionBiologic) in static conditions for 24 h, 60 h, 5, 7 and 10 days at 37°C in triplicate for each condition. The anti-Xa activity in the plasma after each incubation period was measured with a chromogenic anti-Xa assay using the Atellica instrument (Siemens, Germany). Second, the potency of each graft to inhibit the formation of thrombin in pooled human plasma (CRYOcheck, PrecisionBiologic) using the Calibrated Automated Thrombogram^{®} (Stago, Asnières sur Seine, France) was evaluated. For this purpose, grafts were incubated as described above, then put into wells with 300 µL of fresh pool plasma (CRYOcheck). Thrombin generation was then measured as recommended by the manufacturer at 37°C with the PPP reagent (Stago, reference 86193).

The results for the grafts coated with a material of the invention as compared to the comparative systems C1 to C6 are presented on **Figure 4A-B** **and** **C**, respectively. The control patches without heparin will show no anti-Xa increase. The longest anti-Xa effect was observed with PEI-(Hep/Chi)₅-Hep endpoint **(1).** The variation of the results of the invention (coating 1) between Figure 4A and 4B, are due to different time points and different series of experiments. From these data, one can clearly see that the material according to the invention shows a sustained inhibition of thrombin in human plasma up to 5 or 7 days compared to the controls where thrombin is normally generated in all time points. Of note, the inhibition of thrombin generation was effective in all samples of the invention despite initial release of anticoagulant (heparin) during the first 24 h of incubation, as assessed with the anti-Xa assays in the supernatant plasma.

### c) Cell adhesion

The performance of the vascular grafts in recruiting endothelial and smooth muscle cells was evaluated in an *in vitro* cell proliferating assay as described below.

The number of cells was counted semi-quantitatively after 5 days of incubation with smooth muscle cells (SMC) and endothelial cells (EC) as shown under **Figure 5****.** SMC proliferation is important in a degradable material in order to repopulate the vascular wall and to trigger ECM formation. Heparin is known to reduce this activity as seen in Figure 5 for LbL System 1.

Those results support that the endothelial cells are able to adhere rapidly to the multilayer polymer LbL coating when it contains a heparinization substrate comprising at least one pair of alternating layer of poly(allyl amine hydrochloride) (PAH) and poly(sodium 4-styrenesulfonate) (PSS) as an outer layer pair and heparin is functionlized by end-point conjugation to this heparinization substrate.

This finding supports that a rapid and complete endothelialisation can be obtained with a material of the invention which is particularly useful in the context of biodegradable vascular grafts comprising a material of the invention on its surface in contact with blood which will be able to induce the *in vivo* transformation into a neo-vessel.

Altogether, those data support the capacity of a material of the invention to inhibit coagulation and thrombus formation after the implantation of a biocompatible device in contact with blood, in particular vascular grafts but not limited to the aforementioned.

Further, the method of preparation of a material of the invention advantageously provides a method resulting in the biocompatible non-woven fibers being homogeneously covered by the multilayered polymer coating by LbL technique. The homogeneity has been shown not only by optical and fluorescence microscopy but is mainly supported by the fact that there is a strong anti-coagulation response suppotring that essentially all the fibers are coated, thereby preventing coagulation events on microdefects.

The LbL coating of material **(2)** further also allowed the attachment of cells and their proliferation, specifically endothelial cells **(****Figure 5****).** Finally, end-point functionalization and in particular conjugation of heparin improved considerably the performance of devices of the invention (patches) with regards to postponing the pre-thrombin formation, and consequently clot formation.

## Claims

1. A material compatible with blood comprising:
- a support comprising biocompatible non-woven fibers;
- a heparinized multilayer polymer layer-by-layer (LbL) coating of said biocompatible non-woven fibers wherein said multilayer polymer coating comprises at least one layer pair of an anionic polymer layer and a cationic polymer layer and the multilayer polymer layer-by-layer (LbL) coating is functionalized with heparin on-to the outer layer pair of said multilayer polymer coating by end-point functionalization and wherein the anionic polymer is selected from heparin and poly(sodium 4-styrene sulfonate) (PSS) or a mixture thereof and the cationic polymer is selected from chitosan or an analogue thereof and poly(allyl amine hydrochloride) (PAH) or a mixture thereof.

2. A material according to any one of claim 1, wherein the biocompatible non-woven fibers are biodegradable.

3. A material according to any one of claims 1 to 2, wherein the biocompatible non-woven fibers consist of a biodegradable polymer selected from the group consisting of degradable polycaprolactone (PCL), polyglycolic acid (PGA), polylactic acid (PLA), poly(lactic-co-glycolic acid) (PLGA), poly-L-lactide (PLLA), poly(glycerol sebacate) (PGS), polydioxanone (PDO, PDS) or poly-p-dioxanone or PGA-Poly-4-hydroxybutyrate (P4HB) copolymer, degradable polyurethanes fibers, and any combinations or derivates thereof.

4. A material according to any one of claims 1 to 3, wherein the biodegradable biocompatible non-woven fibers comprise or consist of polycaprolactone (PCL) fibers, in particular electrospun ε-PCL nano/microfibers.

5. A material according to any one of claims 1 to 4, wherein the biocompatible non-woven fibers have a diameter of about 0.5 to about 5 µm, such as from about 1 to about 5 µm.

6. A material according to any one of claims 1 to 5, wherein the anionic polymer is heparin.

7. A material according to any one of claims 1 to 7, wherein the cationic polymer is chitosan or an analogue thereof.

8. A material according to any one of claims 1 to 5, wherein the material further comprises an endothelial cell-attracting coating of the multilayer polymer layer-by-layer (LbL) coating onto which heparin is functionalized by end-point functionalization, in particular end-point conjugation.

9. A material according to any one of claims 1 to 8, wherein the biocompatible non-woven fibers are first coated with a pre-coating deposit comprising an adhesion agent for the multilayer polymer coating such as a cationic polymer, in particular poly(ethylene imine) (PEI).

10. A material according to any one of claims 1 to 9, wherein the multilayer polymer LbL coating comprises from about three layer pairs to about eight layer pairs of an anionic polymer layer and a cationic polymer layer as defined in any one of the previous claims, wherein said layer pairs are arranged such that the anionic polymer and the cationic polymer layers are alternating in the multilayer polymer coating.

11. A material according to any one of claims 1 to 10, wherein the multilayer polymer LbL coating comprises from about 3 to about 8, in particular 3 to 5 alternating anionic/cationic polymer layer pairs, wherein the polymer layer pairs are selected from heparin/chitosan or an analogue thereof layer pairs and PAH/PSS layer pairs, or a combination thereof.

12. A material according to any one of claims 1 to 11, wherein the multilayer polymer coating of the biocompatible non-woven fibers comprises at least one pair of alternating layer of poly(allyl amine hydrochloride) (PAH) and poly(sodium 4-styrenesulfonate) (PSS) as an outer layer pair and forms a substrate for end-point functionalization of heparin.

13. A material according to any one of claims 1 to 12 comprising biocompatible non-woven fibers coated with a system comprising a multilayer LbL polymer assembly from the following group:
- **(optional adhesion agent)-(Hep/Chi)₅-Hep,** wherein the last LbL layer of **(Hep/Chi)₅** is functionalized with Heparin by end-point conjugation; and
- **(optional adhesion agent)-(PSS/PAH)₄-Hep,** wherein the last LbL layer of **(PSS/PAH)₄** is functionalized with Heparin by end-point conjugation.

14. A method for preparing a material compatible with blood comprising the steps of:
- providing biocompatible non-woven fibers as a support;
- optionally coating said support with a pre-coating deposit comprising an adhesion agent for a multilayered polymer LbL coating;
- coating said support, pre-coated or not, with a multi-layered polymer LbL coating; wherein the multilayer polymer LbL coating comprises at least one layer pair of an anionic polymer layer and a cationic polymer layer wherein the anionic polymer is selected from heparin and poly(styrene sulfonate) (PSS) or a mixture thereof and the cationic polymer is selected from chitosan or an analogue thereof and poly(allyl amine hydrochloride) (PAH) or a mixture thereof;
- functionalizing the outer layer pair of said multilayer polymer layer-by-layer (LbL) coating with heparin by end-point functionalization.

15. An implantable device comprising a material according to any one of claims 1 to 13, or a material obtainable according to a process of claim 14, such as an artificial blood vessel.
